# EUROPEAN PATENT APPLICATION

(11) **EP 1 792 578 A1**
(43) Date of publication of application: **06.06.2007**
(21) Application number: 06024380.5
(22) Date of filing: 24.11.2006
(51) Int. Cl.: A61B 17/72, A61B 17/17, A61B 17/86, A61B 17/92

(54) **Implant and applicator for osteosynthesis of the elbow**

(30) Priority: 02.12.2005 IT CR20050017
(71) Applicant: LISANTI, Michèle, 56124 Pisa (IT); GARGIULO, Angelo, 84020 Carugo CO (IT)
(72) Inventor: Lisanti, Michele, 56124 Pisa (IT)
(74) Representative: Biazzi, Riccardo

(57) **Abstract**

Implant for the osteosynthesis of the elbow, comprising a nail (1) for entry from the olecranon process, having a double blocking system through distal screws (2) and a proximal traction screw (3) with a spiked washer (4). A suitable applicator for the nail (1) is also disclosed.

## Description

The invention concerns an implant for osteosynthesis of the elbow, and in greater detail for repairing proximal meta-epiphyseal fractures of the ulna. The invention also concerns an applicator for facilitating surgical insertion of said implant.

The elbow is formed of the joint between the distal epiphysis of the humerus, the proximal epiphysis of the ulna and the proximal epiphysis of the radius. Fractures in the region of this joint can seriously and permanently affect the function. Surgical repair of these fractures is often difficult, and involves extensive surgical exposure with risk of infection and failure in healing.

According to a known technique, osteosynthesis of fractures of the long bones (for example, femur, tibia) is made by inserting special nails, called intramedullary nails, in the medullary canal, and locking these nails to the bone using transverse screws (blocked nail).

The nails adapted to repair fractures of the leg or the hip are, however, unsuitable for treatment of the elbow, because they are too big and cannot be applied due to the curved shape of the proximal metaphysis of the ulna.

The purpose of the invention is to make it possible to repair elbow fractures with minimum surgical incisions, obtaining a stable osteosynthesis,

In greater detail, the purpose of the present invention is to achieve stable osteosynthesis of fractures of proximal meta-epiphysis of the ulna by means of closed surgical technique, with entry from the olecranon process, thereby allowing fixation with low trauma, blood loss and risk of infection.

In even greater detail, the invention aims to improve osteosynthesis in the following cases: proximal meta-epiphyseal fractures of the ulna; 3^{rd} proximal fracture of the diaphysis with or without third fragment; bifocal and spiral fractures; pseudoarthrosis and malunions.

The purposes are achieved with an implant for osteosynthesis of the elbow, comprising a nail for entry from the olecranon process, having a proximal part and a distal part, with a double blocking system by means of distal screws that can be inserted in distal holes provided on said distal part of the nail, and a proximal traction screw with spiked washer, that can be inserted in an axial hole provided on said proximal part of nail.

Preferably, the nail is made of grade 5 titanium; the cross section is circular with diameter varying from 6 mm in the proximal part to 5 mm in the distal part; the nail is preferably made available with standard lengths of 80-100-120-140 mm with 5 holes for the distal screws.

The axis of each distal hole is normally perpendicular to that of the nail; according to the invention, however, at least one of said holes may be provided with a 45 deg inclined axis, preferably the closest hole to the proximal part of the nail.

The distal screws, the proximal screw and the spiked washer are also preferably made of titanium. The spiked washer, in particular, is provided with a plurality of triangular shaped hooks on one side, to grip the olecranon while the proximal screw is being screwed in the nail.

The object of the invention also includes a precision applicator for said osteosynthesis implant, comprising a cannulated beater for inserting the nail in the ulna; a precision guide or alignment frame to be fixed to said cannulated beater; cannulated guides associable to said frame for inserting the distal screws with closed-tracing of the distal holes of the nail.

The main feature of the invention is the double blocking system of the osteosynthesis nail, by means of said distal screws and proximal screw-washer assembly, which serves to compress the olecranon. The advantages consist in a stable osteosynthesis, particularly of the fractures of the proximal meta-epiphysis of the ulna, with minimum trauma, minimum blood loss and low risk of infection.

A further advantage is the possibility of compression of the centre of the fracture, when necessary, by means of the proximal traction screw. Said screw can, in fact, enter the proximal hole of the nail by many millimetres and therefore, if the nail is blocked first by means of a distal screw, the tightening of the proximal screw will bring the proximal fragment closer to the distal fragment, with a strong grip on the bone by means of the spiked washer.

The invention is now described in detail, with reference to a preferred embodiment as an example, with the help of enclosed figures wherein:
Fig. 1 illustrates an osteosynthesis implant for the elbow, according to the invention;
Fig. 2 refers, in particular, to the nail of the implant of Fig. 1;
Fig, 3 illustrates the spiked washer and proximal traction screw of the implant of Fig. 1;
Fig. 4 illustrates, in a schematic manner, the cannulated beater for inserting nail of Fig. 1 in the ulna;
Fig. 5 illustrates, in a schematic manner, a kit for surgical application of the implant of Fig. 1;
Fig. 6 is an X-ray view of an implant according to the invention inserted into the human body.

The figures illustrate an osteosynthesis implant for the elbow, in particular for repairing proximal meta-epiphyseal fractures of the ulna, The implant comprises a nail 1, one or more distal screws 2, a proximal or top screw 3, a spiked washer 4.

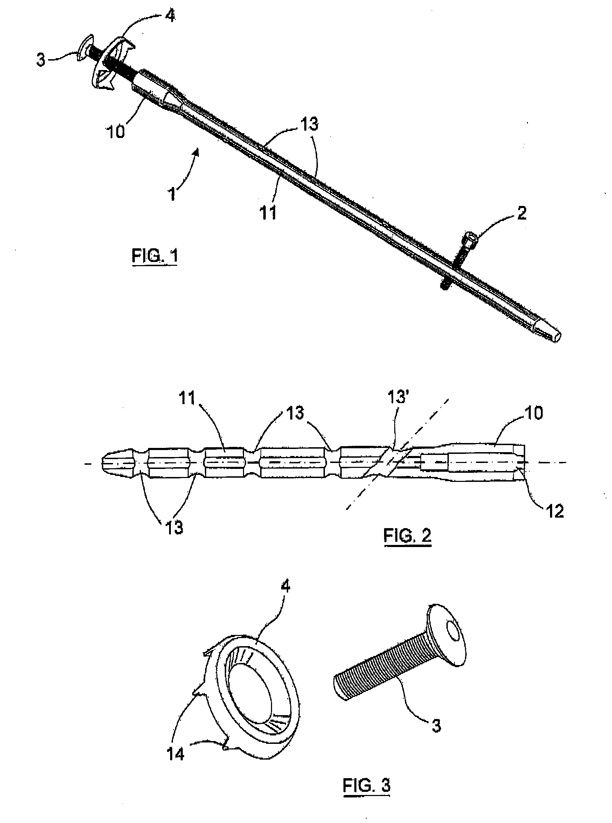

In greater detail, nail 1 (Fig. 2) is a cannulated nail for insertion from the olecranon process, and comprises a proximal or top part 10 and a distal part 11. The proximal part 10 has an axial threaded hole 12, for insertion of proximal screw 3; the distal part 11, on the other hand, comprises transverse holes 13 for insertion of the distal screws 2, with axis perpendicular to that of the nail 1.

Preferably, at least one hole 13' is also provided, with axis inclined at 45 degrees with respect to the nail axis; said inclined hole 13' is useful, in some cases, to fix by screw certain unstable interposed fragments, or when compression is not desired (danger of shortening if a fracture of the ulna is associated with a fracture of the radius), for stabilization with a proximal grip on the olecranon bone.

Said inclined hole 13' is preferably the first hole closest to the top part 10 of nail 1, as is seen in Fig. 2. The distance from the centre of the hole to the proximal is, for example, 25 mm.

According to a preferred embodiment, nail 1 is made of grade 5 titanium; the cross section is preferably circular, with a 6 mm diameter at the top part 10, and a 5 mm diameter at the distal part 11.

Nail 1 is preferably realized with standard lengths of 80-100-120-140 mm; the distal part 11 preferably comprises five holes 13, 13' for screws 2; the diameter of said holes is 2.9 mm.

The spiked washer 4 (Fig. 3) is provided with a plurality of teeth or hooks 14, preferably triangular, to allow for a firm grip on the olecranon.

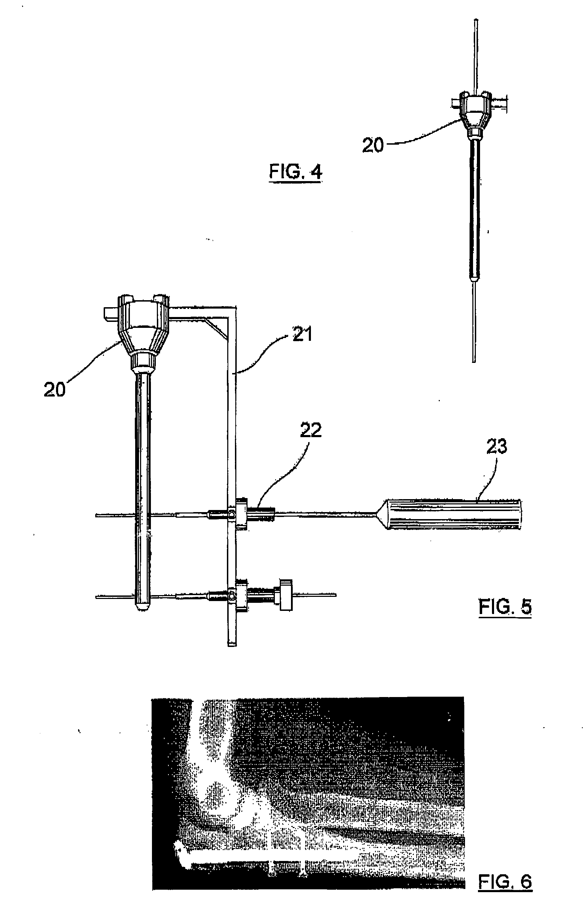

Fig. 4 and 5 illustrates, by way of example, a kit for the application of the implant of Fig. 1. Basically, the kit comprises a cannulated beater 20 (Fig. 4) for inserting nail 1 in the ulna, a precision guide or alignment frame 21 and cannulated guides 22, that can be fixed to said frame 21 for insertion of distal screws 2 (Fig. 5).

The frame 21 can be screwed to the beater 20 and allows closed tracing of distal holes of the nail 1. The figure also illustrates a suitable screwdriver 23 in position for fixing the distal screws 2.

The surgical implantation technique, by way of example, can be as follows.

The patient is made to lie flat on the orthopaedic table, with the arm to be operated on the side. The beater 20 is fitted on top of the nail 1, without mounting the frame 21 for the distal screws. Radioscopy is carried out to check the exact position of the fracture(s) and the skin is cut.

A guide wire is introduced to pass through the fracture and to reach the diaphyseal canal a few centimetres beyond the fracture. Radioscopy is carried out to check the position of the guide wire. If the guide wire has gone beyond the fracture rim, the ulna is milled to the selected nail length, taking care to avoid damaging the walls of the bone.

After this, the olecranon is milled to form a small seat, so that the proximal part 10 of nail 1, which has a greater diameter than the distal part 11, can easily pass through. Nail 1 is then inserted on the guide wire.

Nail 1 must be carefully pushed into the ulna, until the proximal part 10 is projecting by 5-6 mm beyond the proximal cortex of the olecranon. When done, frame 21 is mounted on beater 20.

Distal screws 2 are then inserted with the following procedure, select the hole where the distal screw is to be positioned; position guide 22 in the selected hole of the frame 21; drill up to the second cortex using a Ø2 mm drill; calculate the length of the screw to be used with normal Kirschner wire; remove the guide and mount the guide for distal screws on the screwdriver; screw the selected screw using the suitable screwdriver up to the second cortex.

Normally, one distal screw is far enough to prevent rotation movements but, if necessary, additional screws can be applied by repeating the steps described above.

Then, the surgeon dismantle the frame 21 from beater 20 and the beater 20 from the nail 1.

Proximal fixation is done by inserting the compression washer 4 in the traction screw 3 and screwing the traction screw 3 in the nail 1, so that the compression washer 4 is locked on the olecranon. See Fig. 6 for an example.

The instrument used for insertion can also be used for extraction. All the screws must be removed before removing the locking nail. In some cases, it may be necessary to clean the thread in the proximal part of the nail to allow insertion and fixation of the extractor.

## Claims

1. Implant for osteosynthesis of the elbow, comprising a nail (1) for entry from the olecranon process, having a proximal part (10) and a distal part (11), with a double blocking system by means of distal screws (2) that can be inserted in distal holes (13, 13') provided on said distal part (11) of the nail (1), and a proximal traction screw (3) with spiked washer (4), that can be inserted in an axial hole (12) provided on said proximal part (10) of the nail (1).

2. Implant according to claim 1, **characterized in that** distal holes of the nail (1) comprises transverse holes (13) with axis perpendicular to the axis of the nail (1) itself, and at least one inclined hole (13') with axis inclined by 45 degrees relative to the axis of said nail (1).

3. Implant according to claim 1 or 2, **characterized in that** the nail (1) has a circular cross section; the proximal part (10) has a diameter of about 6 mm; the distal part (11) has a diameter of about 5 mm.

4. Implant according to any of the preceding claims, **characterized in that** nail (1) has a length that can be selected from values of 80, 100, 120 and 140 mm.

5. Implant according to any of the preceding claims, **characterized in that** the nail (1) has five distal holes (13, 13') for insertion of said distal screws (2) in predetermined positions.

6. Implant according to any of the preceding claims, **characterized in that** the washer (4) has a plurality of hooks (14), on one side, suitable for gripping the olecranon.

7. Implant according to claim 6, **characterized in that** said hooks (14) of the washer (4) consist of triangular teeth.

8. Implant according to any of the preceding claims, **characterized in that** nail (1), screws (2, 3) and washer (4) are made of grade 5 titanium.

9. A kit for the application of an osteosynthesis implant according to any one of preceding claims, said kit comprising a cannulated beater (20) for inserting the nail (1) in the ulna; an alignment frame (21) which can be fixed to said beater (20); cannulated guides (22) which can be fixed to said frame (21) for inserting the distal screws (2) with closed-tracing of the holes (13, 13') of said nail (1).
